# EUROPÄISCHE PATENTSCHRIFT

(11) **EP 2 533 771 B1**
(45) Veröffentlichungstag und Bekanntmachung des Hinweises auf die Patenterteilung: **18.09.2019**
(21) Anmeldenummer: 10790895.6
(22) Anmeldetag: 02.12.2010
(51) Int. Cl.: A61K 9/70, A61K 38/24, A61P 5/06

(54) **TRANSDERMALES THERAPEUTISCHES SYSTEM FÜR DIE VERABREICHUNG VON PEPTIDEN**
TRANSDERMAL THERAPEUTIC SYSTEM FOR THE ADMINISTRATION OF PEPTIDES
SYSTÈME THÉRAPEUTIQUE TRANSDERMIQUE POUR L'ADMINISTRATION DE PEPTIDES

(30) Priorität: 04.12.2009 DE 102009056745
(43) Veröffentlichungstag der Anmeldung: 19.12.2012
(73) Patentinhaber: LTS LOHMANN Therapie-Systeme AG, 56626 Andernach (DE)
(72) Erfinder: HORSTMANN, Michael, 56564 Neuwied (DE); HOFFMANN, Gerd, 56566 Neuwied (DE); WIEDERSBERG, Sandra, 06268 Albersroda (DE)
(74) Vertreter: Schweitzer, Klaus
(86) Internationale Anmeldenummer: PCT/EP2010/007323
(87) Internationale Veröffentlichungsnummer: WO 2011/066971

(56) Entgegenhaltungen:
- WO-A1-01/80859
- WO-A2-2004/039426
- DE-A1-102007 006 244
- DE-U1-202006 000 662
- US-A1- 2003 147 943

## Beschreibung

Der Gegenstand der vorliegenden Erfindung ist die Verwendung mindestens eines Peptids zur Herstellung eines Transdermalen Therapeutisches Systems (TTS) zur Verabreichung dieses Peptids durch ablativ behandelte Haut an einen Patienten, wobei es sich bei diesem Peptid um das follikelstimulierende Hormon (Follitropin, FSH) oder das somatotrope Hormon (Somatotropin, STH) handelt.

Transdermale Therapeutische Systeme (TTS) sind seit langem als pharmazeutische Darreichungsformen bekannt. Für die transdermale Applikation von pharmazeutischen Wirkstoffen mittels TTS stellt das Stratum corneum (SC), die äußerste Schicht der Haut, in den meisten Fällen die eigentliche Barriere für die Durchlässigkeit und die Geschwindigkeit des Durchtritts des pharmazeutischen Wirkstoffs dar.

Peptide und Proteine sowie andere hochmolekulare Moleküle mit einem Molekulargewicht oberhalb von 500 Dalton - wie beispielsweise Tacrolimus, Heparin und zahlreiche Salze von Betamethason - werden aufgrund ihrer Molekülgröße und ihrer physiko-chemischen Eigenschaften im Allgemeinen transdermal nicht resorbiert.

Die meisten Peptide besitzen zudem eine geringe orale Bioverfügbarkeit und unterliegen einem starken, proteolytischem Abbau im Gastrointestinaltrakt. Daher werden Peptide gewöhnlich parenteral, unter Umgehung des Gastrointestinaltraktes, verabreicht. Hierbei handelt es sich um Injektionen oder Infusionen die unter die Haut, in den Muskel oder direkt in die Blutbahn appliziert werden.

Die transdermale Route würde hier eine nichtinvasive Alternative mit hoher Patientencompliance zu dieser invasiven, parenteralen Verabreichung bieten. Es gibt daher zahlreiche Ansätze, die Durchlässigkeit der Haut für Moleküle mit einem Molekulargewicht oberhalb von 500 Dalton zu erleichtern. Hierzu zählen in erster Linie der Einsatz von Permeationsverstärkern ("Enhancern") oder die zusätzliche Anwendung von Wärme.

Eine weitere Methode schlecht hautgängige Moleküle für die transdermale Verabreichung zugänglich zu machen besteht darin, den Durchgang eines solchen Wirkstoffs durch das Stratum corneum zu erleichtern, indem diese Schicht zuvor teilweise zerstört oder entfernt wird. Diese als "skin ablation" bezeichneten Techniken verwenden thermische oder mechanische Energie, um das Stratum corneums partiell zu zerstören oder zu entfernen und so direkte Kanäle in die lebende Epidermis zu schaffen. Die Durchlässigkeit der Haut wird erhöht und eine transdermale Resorption von hochmolekularen Molekülen kann somit ermöglicht werden.

Durch diese Vorbehandlung der Haut können zudem auch hydrophile Wirkstoffe transdermal verabreicht werden, die bisher aufgrund ihrer Hydrophilie der transdermalen Route verschlossen waren. Hier kommen beispielsweise Fentanylcitrat, Granisetron-HCl, Na-Diclofenac und Apomorphin-Sulfat in Frage. Weiterhin kann die TTS-Fläche bestehender TTS Systeme durch eine "skin ablation" Vorbehandlung der Haut bei gleichen Blutspiegeln signifikant reduziert werden.

Gewöhnlich wird durch die "skin-ablation-Technik" eine Vielzahl von Mikrokanälen durch das Stratum Corneum erzeugt, jedoch ist der prozentuale "gelöcherte" Anteil der behandelten Hautfläche relativ gering. Eine Beschreibung der "skin-ablation-Technik" mittels Laser ist in WO 2007/039646 enthalten.

Das Follikel-stimulierende Hormon (FSH, Follitropin) ist ein gonadotropes Hormon des Hypophysen-Vorderlappens, das auch Follikelreifungshormon, Gonadotropin A, Prolan A oder Thylakentrin genannt wird. Menschliches FSH ist ein saures Glycoprotein (isoelektrischer Punkt 4,5) mit 16 % Kohlenhydrat-Anteil und einem Molekulargewicht von etwa 34.000 Dalton. Seine α-Polypeptid-Kette (mit 92 Aminosäure-Resten) ist nahezu identisch mit derjenigen von Chorio(n)gonadotrop(h)in. Die für FSH spezifische β-Kette enthält 111 Aminosäure-Reste. FSH fördert Wachstum und Entwicklung der Keimdrüsen (Gonaden) und regt diese zur Hormon-Synthese an. Bei Frauen spielt es im Menstruationszyklus eine Rolle, indem es die Reifung eines neuen Follikels und dessen Produktion von Estradiol bewirkt. In den männlichen Keimdrüsen stimuliert es die Bildung von Samenzellen. Für die hier beschriebene Verwendung kann FSH aus natürlichen Quellen oder rekombinantes FSH eingesetzt werden.

FSH hat eine relativ kurze Halbwertzeit. Bei der Superovulation von Wiederkäuern sind vielfach Hypophysenextrakte, die dann allerdings neben FSH auch unterschiedliche Mengen an LH (luteinisierendes Hormon) enthalten können, eingesetzt worden. Heute sind auch rekombinante FSH-Präparate kommerziell erhältlich (Gonal F®, Puregon®).

Somatotropin (auch: somatotropes Hormon, STH, GH) ist ein im Hypophysen-Vorderlappen gebildetes, tierartspezifisches Hormon, das für den Wachstumsprozess verantwortlich ist. Das auch HGH (hypophyseal oder human growth hormone) genannte Wachstumshormon des Menschen ist ein einzelnes Polypeptid, Molmasse ca. 21.500, aus 191 Aminosäuren mit 2 Disulfid-Brücken. Menschliches Somatotropin ist in seiner Zusammensetzung eng verwandt mit Placentalactogen sowie mit Prolactin. Somatotropin bewirkt in Leber und Niere die Ausschüttung von Insulin-artigen Wachstumsfaktoren, die für einen Großteil der Wirkungen des Somatotropin verantwortlich sind. Gehemmt wird die Somatotropin-Sekretion durch Somatostatin, stimuliert wird sie durch das Releasing-Hormon Somatoliberin (SRF oder SRH bzw. GH-RF oder GH-RH) aus dem Hypothalamus.

Durch Fehlen oder Unterproduktion von Somatotropin bei Kindern bewirkter Zwergwuchs kann durch Zufuhr menschlichen Wachstumshormons reguliert werden, das in den USA inzwischen gentechnisch hergestellt wird als Protropin® (Genentech) und - mit einem unterschiedlichen Aminosäure-Rest - Humatrope® (Eli Lilly). Weitere medizinische Anwendungen für Somatotropin könnten sich bei Verbrennungen, Alterserscheinungen, Osteoporose, Herz-Kreislauf-Erkrankungen und Fettleibigkeit ergeben.

Die bekannten Erzeugnisse besitzen jedoch einige Nachteile, die insbesondere auf die allgemein bekannte geringe Stabilität der Peptide in Lösung zurückzuführen sind.

Hochmolekulare Moleküle sind der transdermalen Applikation bislang aufgrund ihrer physiko-chemischen Eigenschaften verschlossen. Erst durch eine Vorbehandlung der Haut wird eine transdermale Applikation dieser Moleküle ermöglicht.

Schließlich können bei der Injektion selbst Schwierigkeiten auftreten, die hauptsächlich in Schmerzen bei der Anwendung, einem Verletzungsrisiko und dem Risiko von Infektionen bestehen.

Aus der DE 10 2007 006244 A1 ist ein TTS zur Verabreichung wasserlöslicher Wirkstoffe wie Somatotropin bekannt, das unter okklusiven Bedingungen verabreicht wird.

Die WO 2004/039426 A2 offenbart ein System zur transdermalen Verabreichung von wasserlöslichen Wirkstoffen wie Steroiden. Die Haut wird vor Applikation des TTS ablativ behandelt.

Aufgabe der vorliegenden Erfindung ist es, ein Transdermales Therapeutisches System (TTS) für die Verabreichung von Peptiden und anderen schlecht hautgängigen Molekülen zur Verfügung zu stellen.

Damit das TTS bei Raumtemperatur lagerstabil ist und wenig mikrobiell anfällig, sollte es möglichst wenig Wasser enthalten.

Dabei soll das TTS auf ein Hautareal aufgebracht werden, von dem zuvor mindest ein Teilbereich des Stratum corneums zerstört oder entfernt wurde.

Es soll insbesondere ein TTS mit dem Wirkstoff Follitropin (FSH; follikelstimulierendes Hormon) und/oder eines seiner pharmazeutisch akzeptablen Salze hergestellt werden, mit dem dieses Peptid durch die Haut in therapeutischen Dosen an einen Patienten verabreicht werden kann.

Bei der Haut soll es sich vorzugsweise um "ablativ" vorbehandelte Haut handeln, bei der ein Anteil des Stratum Corneum entfernt worden ist.

Dabei soll nicht nur der Weg der Verabreichung mittels Injektion vermieden werden. Auch das TTS selbst soll möglichst ohne Mikroinjektionsnadeln, Mikromesser und / oder sonstige Nadeln und Widerhaken ausgestattet sein, um eine zusätzliche mechanische Verletzung des Stratum corneums zu vermeiden bzw. auszuschließen. Es kann aber gegebenenfalls mit derartigen Konstruktionselementen ausgerüstet sein.

Auch soll das Peptid im Rahmen einer Langzeitanwendung mit Hilfe des Transdermalen Therapeutischen Systems appliziert werden können.

Auch soll das Erzeugnis auf einfache und kostengünstige Weise herstellbar sein.

Gelöst wird die Aufgabe durch die Verwendung mindestens eines Peptids zur Herstellung eines Transdermalen Therapeutisches Systems (TTS), umfassend eine Rückschicht, die mit einer haftklebenden Schicht aus mindestens einem nicht-wasserlöslichen Polymer ausgerüstet ist, eine wirkstoffhaltige Schicht, die mindestens dieses Peptid und eine Trägersubstanz in Form eines textilen Flächengebildes enthält, und eine Schutzfolie, worin es sich bei dem Peptid um das follikelstimulierende Hormon (Follitropin, FSH) oder das somatotrope Hormon (Somatotropin, STH) handelt und die wirkstoffhaltige Schicht eine Fläche von 1 bis 100 cm², vorzugsweise von 2 bis 80 cm² sowie eine Dicke zwischen 10 und 200 µm, vorzugsweise zwischen 15 und 90 µm und besonders bevorzugt zwischen 20 und 80 µm aufweist, zur Verabreichung dieses Peptids durch ablativ behandelte Haut an einen Patienten.

Das TTS kann weiterhin eine Rückschicht enthalten, die für das Peptid undurchlässig ist. In einer bevorzugten Ausführungsform ist die Rückschicht auf der, der wirkstoffhaltigen Schicht zugewandten Seite mit einem nicht-wasserlöslichen, haftklebenden Polymer beschichtet. Vorzugsweise besitzt eine solche Rückschicht eine Fläche, die größer ist als die Fläche der wirkstoffhaltigen Schicht. In einem solchen TTS bildet die Rückschicht ein "Überpflaster", das für die sichere Haftung des TTS auf der Haut sorgt.

Die wirkstoffhaltige Schicht kann weitere, den Wirkstoff stabilisierende Hilfsstoffe enthalten, vorzugsweise Puffersubstanzen oder Zucker, aber auch Stabilisatoren und Konservierungsmittel.

Das TTS kann auch mindestens eine weitere zusätzliche Haftkleberschicht enthalten, die im Wesentlichen frei von Wirkstoff und haftklebend ist. Eine solche zusätzliche Haftkleberschicht sorgt in dem Fall, dass die wirkstoffhaltige Schicht nicht oder nicht ausreichend genug haftklebend ist, für eine sichere Haftung des TTS auf der Haut.

In einer besonderen Ausführungsform enthält das TTS den Wirkstoff Follitropin und/oder mindestens eines seiner pharmazeutisch akzeptablen Salze.

In einer weiteren besonderen Ausführungsform enthält das TTS den Wirkstoff Somatotropin.

Ein "Transdermales Therapeutisches System" (TTS) ist ein schichtförmig aufgebautes Erzeugnis. In seiner einfachsten Ausführungsform besteht es aus einer Rückschicht, einer wirkstoffhaltigen Schicht und einer Schutzfolie, die die wirkstoffhaltige Schicht bis zur Anwendung des TTS abdeckt. Bei einer solch einfachen Konstruktion ist die wirkstoffhaltige Schicht vorzugsweise haftklebend ausgerüstet. Wenn die Klebkraft der wirkstoffhaltigen Schicht jedoch ungenügend ist, kann das TTS eine zusätzliche Haftkleberschicht aufweisen.

Diese zusätzliche Haftkleberschicht kann zwischen der wirkstoffhaltigen Schicht und der Schutzfolie angeordnet sein.

In einer bevorzugten Ausführungsform ist die zusätzliche Haftkleberschicht zwischen der wirkstoffhaltigen Schicht und der Rückschicht angebracht. In diesem Fall überragt die Haftkleberschicht die wirkstoffhaltige Schicht an zumindest einem Abschnitt entlang des/der seitlichen Rands/Ränder der wirkstoffhaltigen Schicht. Die zusätzliche Haftkleberschicht sorgt dann als "Überpflaster" während der Anwendung des TTS für eine sichere Haftung auf der Haut.

Das TTS kann auch eine Membran besitzen, die die Geschwindigkeit des Austritts des Wirkstoffs aus der wirkstoffhaltigen Schicht kontrolliert. Die Membran ist daher auf der Seite der wirkstoffhaltigen Schicht angebracht, die während der Anwendung des TTS der Haut zugewandt ist.

Das TTS selbst kann schließlich eine Nadelschicht besitzen, die direkt mit der Haut in Kontakt kommt und an seiner Unterseite mit Mikroinjektionsnadeln (= Hohlnadeln für den Durchfluss von Wirkstoff), Mikromessern (zum Anritzen der obersten Hautschichten), Nadeln (zum Perforieren der obersten Hautschichten) und/oder Widerhaken (zur Verankerung in der Haut) ausgestattet ist. In einer bevorzugten Ausführungsform ist das TTS jedoch ohne eine derartige Schicht ausgestattet.

In einer weiteren Ausführungsform kann das Transdermale Therapeutische System mehr als eine wirkstoffhaltige Schicht enthalten. Diese wirkstoffhaltigen Schichten können übereinander (wobei ein mindestens zweischichtiges Laminat gebildet wird) oder nebeneinander angeordnet sein. Im Fall eines solchen TTS mit mehr als einer wirkstoffhaltigen Schicht können die einzelnen Schichten gleich oder unterschiedlich aufgebaut sein. In derartigen "mehrschichtigen Systemen" unterscheiden sich diese Schichten jedoch vorzugsweise aufgrund ihrer Zusammensetzung oder des verwendeten Wirkstoffs. Auch kann die wirkstoffhaltige Schicht in Form eines mit Flüssigkeit gefüllten Beutels bzw. einer mit Flüssigkeit gefüllten Kammer vorliegen, in der der Wirkstoff in gelöster, dispergierter oder suspendierter Form enthalten ist.

Schließlich kann der Wirkstoff in der wirkstoffhaltigen Schicht in flüssigen Mikroreservoiren enthalten sein, welche in der wirkstoffhaltigen Schicht dispergiert sind.

Mit dem hier beschriebenen TTS können die Peptide follikelstimulierendes Hormon (Follitropin, FSH) oder somatotropes Hormon (Somatotropin, STH) als Wirkstoff auf transdermalem Wege verabreicht werden. Die technische Lehre kann aber grundsätzlich auch für andere physiologisch wirksame Substanzen genutzt werden, insbesondere auch solche, die bisher der transdermalen Therapie noch nicht zur Verfügung stehen (hydrophile Wirkstoffe) oder eine Molekularmasse oberhalb von 500, vorzugsweise oberhalb von 1.500 Dalton besitzen.

Unter "Haut" ist die normale, intakte Haut eines Menschen oder Säugetiers zu verstehen. Diese ist schichtförmig aufgebaut und besteht - von außen nach innen gesehen - aus Epidermis (Oberhaut), Dermis (Lederhaut) und Subcutis (Unterhaut). Innerhalb dieser drei Bestandteile werden vom Fachmann ggf. weitere Schichten unterschieden.

Bei der Epidermis werden fünf Schichten unterschieden: Hornschicht (Stratum corneum), Glanzschicht (Stratum lucidum), Körnerschicht (Stratum granulosum), Stachelzellschicht (Stratum spinosum) und Basalschicht (Stratum basale).

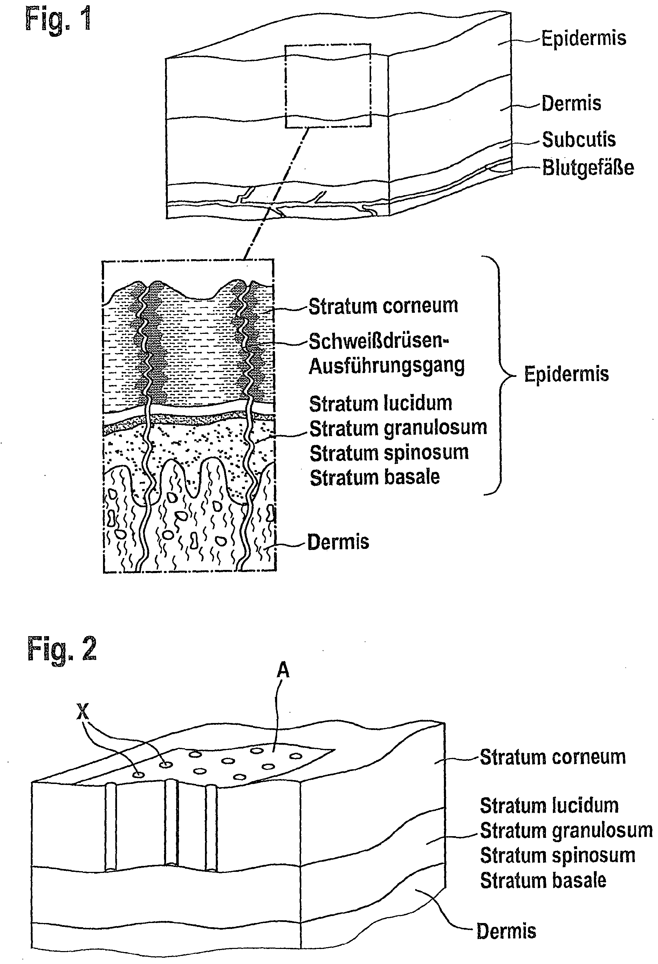

Unter "ablativ behandelter Haut" ist die normale, intakte Haut eines Menschen zu verstehen, von deren Epidermis das Stratum corneum - zumindest teilweise - zerstört oder entfernt worden ist. Dabei kann in diesem Bereich der ablativ behandelten Haut der "Flächenanteil von normaler, intakter Haut, von deren Epidermis das mindestens das Stratum corneum zerstört oder entfernt ist" (entspricht der Summe der Flächen X in Abb. 2) zur "gesamten normalen, intakten Haut, an deren Epidermis das Stratum corneum verbleibt" (entspricht der Fläche A in Abb. 2), unterhalb von 50% liegen, vorzugsweise unterhalb von 20% und besonders bevorzugt unterhalb von 10%. Die Abschnitte der Epidermis, an denen das Stratum corneum entfernt wurde, können unregelmäßig geformt sein. Vorzugsweise sind sie aber von definierter Form und Fläche. Als geeignete Formen kommen Rechtecke, Sechsecke, Achtecke, Quadrate, Kreise und Punkte in Frage. Die Abschnitte der Epidermis, die durch ablative Behandlung entfernt werden, sind so tief, dass an den betreffenden Stellen mindestens das Stratum corneum entfernt wird und so die "Mikrokanäle" unter den Flächen X (vgl. Abb. 2) entstehen. Die durch ablative Behandlung entfernten Abschnitte der Epidermis sollen vorzugsweise aber nicht tiefer reichen als bis zur Dermis. Dies kann durch eine entsprechende Anpassung der Leistung des Lasers und zeitgleich erfolgende Kontrollmessungen erreicht werden.

Als "transdermal" wird die Applikationsroute durch die Haut eines Menschen oder Säugetiers bezeichnet. Dabei ist unter Haut sowohl die normale, intakte Haut sowie die "ablativ behandelte Haut" im Sinne der vorstehenden Definition zu verstehen.

Als "Trägersubstanz" für die wirkstoffhaltige Schicht kommen Substanzen in Frage, die sich in Bezug auf das mindestens eine Peptid kompatibel verhalten. Es ist bekannt, dass sich bei Peptiden sowohl durch chemische Einflüsse, wie zum Beispiel Säuren, Salze oder organische Lösungsmittel, als auch durch physikalische Einwirkungen, wie hohe oder tiefe Temperaturen oder auch Druck, die Sekundär- und Tertiärstruktur und damit letztlich auch die Quartärstruktur verändern können (Denaturierung). Durch eine Denaturierung können sich auch die physikalischen und physiologischen Eigenschaften der Peptide ändern. Bei einer chemischen Spaltung der Peptide (Proteolyse) entstehen daraus Teilstücke, die man Peptone nennt.

Das bedeutet hinsichtlich der Anforderungen an die Kompatibilität der Trägersubstanz, dass beim Einbetten des Peptids in die Trägersubstanz keine Wechselwirkung mit dem Peptid auftreten dürfen, die zu einer solchen Veränderung der Struktur des Peptids oder zu einer auf anderen Ursachen beruhenden Verschlechterung seiner pharmakologischen Eigenschaften führen.

Die Trägersubstanz bewirkt, dass das mindestens eine Peptid in der wirkstoffhaltigen Schicht gleichmäßig verteilt ist. Vorzugsweise bewirkt die Trägersubstanz, dass die Peptidmoleküle einzeln vorliegen, d.h. in Form einer echten "Lösung".

Es hat sich herausgestellt, dass insbesondere solche Trägersubstanzen geeignet sind, die "hydrophil" sind. Unter hydrophil ("wasserliebend") versteht man die Fähigkeit, Wasser an sich zu binden bzw. in Wasser einzudringen und in einem weiteren Sinne "von Wasser gut benetzt zu werden".

Die Trägersubstanz kann in der wirkstoffhaltigen Schicht in Form von Fasern, Pulver oder als Film vorliegen. Bevorzugt bildet die Trägersubstanz ein Film mit einer konstanten Schichtdicke. Diese Schichtdicke kann zwischen 20 und 200 µm liegen, vorzugsweise zwischen 30 und 80 µm.

In einer weiteren bevorzugten Ausführungsform liegt die Trägersubstanz als textiles Flächengebilde vor, vorzugsweise als Vliesstoff aus einzelnen Fasern, aber auch in Form eines Gewebes, eines Gestrickes oder eines Gewirkes aus Garn. In diesen Fällen ist die Trägersubstanz nicht wasserlöslich.

Die wirkstoffhaltige Schicht kann "Puffer" enthalten, um darin einen definierten pH-Wert aufrecht zu erhalten und die Stabilität des Wirkstoffs zu erhöhen. Puffersysteme und die damit einstellbaren pH-Werte sind dem Fachmann bekannt. Für FSH ist ein Puffer, der einen pH-Wert von ungefähr 7 gewährleistet, bevorzugt.

Als "Rückschicht" kommen okklusive und nicht-okklusive Schichten in Frage, wobei okklusive bevorzugt sind. Diese Schichten sind aus Folien, Geweben und/oder Gewirken aufgebaut, wobei Folien bevorzugt sind. Bei den Materialien handelt es sich um natürliche oder synthetische Polymere und Metalle. Besonders bevorzugt sind Verbundwerkstoffe aus synthetischen Polymeren und Metallen in Form von Laminaten. Die Rückschicht ist vorzugsweise flexibel und für den Wirkstoff undurchlässig.

Die "wirkstoffhaltige Schicht" enthält - wie bereits gesagt - mindestens ein Peptid und mindestens eine Trägersubstanz für das Peptid. Sie kann eine Fläche von 1 bis 100 cm² aufweisen, vorzugsweise von 2 bis 80 cm² und besonders bevorzugt zwischen 4 bis 20 cm². Die Schichtdicke der wirkstoffhaltigen Schicht kann zwischen 10 und 200 µm, vorzugsweise zwischen 15 und 90 µm liegen, besonders bevorzugt zwischen 20 und 80 µm.

Die "Konzentration" des mindestens einen Peptids in der wirkstoffhaltigen Schicht hängt stark von der therapeutischen Indikation, der Aktivität des jeweiligen Peptids und dessen Molekulargewicht ab. Die Konzentration kann daher in weiten Bereichen variieren und in der wirkstoffhaltigen Schicht zwischen 0,1 bis 99 Gew.-%, vorzugsweise zwischen 30 und 70 Gew.-% liegen.

Damit die wirkstoffhaltige Schicht, die ein Peptid und eine Trägersubstanz für das Peptid enthält, "haftklebend" ausgerüstet ist, kann ihr mindestens ein "Haftkleber" zugegeben werden. Die geeigneten Haftkleber sind weiter unten aufgeführt. Eine andere Möglichkeit besteht darin, durch Zugabe von Weichmachern, Tackifiern etc., die wirkstoffhaltige Schicht haftklebend auszurüsten. Insbesondere, wenn die Trägersubstanz stark hydrophil ist, ist die Verwendung von hydrophilen Klebrigmachern wie Pantothenylalkohol, Honig, niedermolekularen Kohlenhydraten (wie Saccharose, Glucose, Fructose) und deren Derivaten (wie beispielsweise Saccharoseacetatisobutyrat) und deren Kombinationen vorteilhaft.

Die wirkstoffhaltige Schicht kann in einer besonderen Ausführungsform Wasser enthalten. Vorzugsweise ist der Wassergehalt (Restfeuchtigkeitsgehalt) jedoch gering, um die mechanische Stabilität der wirkstoffhaltigen Schicht nicht zu gefährden und andere - insbesondere mikrobiologische - Risiken aufgrund der Anwesenheit von Wasser zu minimieren. Bevorzugt ist der "Wassergehalt" in der wirkstoffhaltigen Schicht unterhalb von 20%, vorzugsweise unterhalb von 10% und besonders bevorzugt unterhalb von 5%.

Die zusätzliche "haftklebende Schicht" kann aus den dem Fachmann bekannten "Haftklebern" aufgebaut sein. Haftkleber sind in der Lage, bei Raumtemperatur ohne eine Aktivierung durch Lösungsmittel oder Wärme lediglich durch Andrücken an die Oberfläche des zu beklebenden Gegenstands eine "Benetzung" herbeizuführen, die ausreichende Haftungskräfte ergibt.

Als "Haftkleber" können "Polymere" eingesetzt werden, die aufgrund der Zusammensetzung ihrer Monomeren haftklebende Eigenschaften besitzen. Hierzu zählen Synthese- und Naturkautschuk, Butylkautschuk, Styrol-Butadien-Copolymere, Ethylen-VinylacetatCopolymere, Acrylnitril-Copolymere, Polychloropren, Polyisobutylen, Polyvinylether, Styrol-Butadien-Styrol-Blockpolymere, Styrol-Isopren-Styrol-Blockpolymere, Polyacrylate, Polyester, Polyurethane und Polysiloxane. Durch funktionelle Gruppen in den Monomeren dieser Polymere können die Klebereigenschaften des bei der Polymerisation erhaltenen Polymers modifiziert werden. Diese Polymere sind nicht-wasserlöslich.

Eine weitere Möglichkeit, die Klebereigenschaften dieser genannten Polymere zu modifizieren bietet die Anpassung der Kleberrezeptur an die gewünschten Eigenschaften durch Zugabe von Zusatzstoffen wie Harzen, Weichmachern, Klebrigmachern, Füllstoffen und / oder Stabilisatoren.

Besonders gut geeignete Polymere mit haftklebenden Eigenschaften sind Polyacrylate, Polyisobutylene, Silikone.

Vorzugsweise werden solche Haftkleber verwendet, die sich durch ihre gute Materialverträglichkeit gegenüber den Peptiden auszeichnen und zugleich keine Hautirritationen, Allergien oder Sensibilisierung bei der Anwendung auslösen.

Als "Schutzfolie" können in dem Transdermalen Therapeutischen System die dem Fachmann bekannten Folien eingesetzt werden, wie z. B. silikonisierte Polyesterfolien.

Die Verwendung des Transdermalen Therapeutischen Systems (TTS), welches eine wirkstoffhaltige Schicht mit mindestens einem Peptid und mindestens einer Trägersubstanz für das Peptid enthält, ist eine weitere erfindungsgemäße Lösung.

Zu diesem Zweck wird vor der Applikation des TTS die Hornschicht (das Stratum corneum) der Haut zumindest abschnittsweise entfernt, vorzugsweise mittels der skin-ablation-Technik mittels Laser. In einer bevorzugten Ausführungsform weist diese ablativ behandelte Haut in diesem Areal Mikrokanäle innerhalb des Stratum corneums auf.

Durch anschließende Applikation des TTS wird die transdermale Resorption des Peptids ermöglicht. Dazu wird das TTS direkt auf die ablativ behandelte Haut platziert. Die wirkstoffhaltige Schicht, die das Peptid und eine Trägersubstanz für das Peptid enthält, kommt dabei direkt oberhalb der ablativ behandelten Haut zum liegen.

Aufgrund der zumindest punktuellen Entfernung des Stratum corneums kann das Peptid die darunter liegenden Hautschichten erreichen und schließlich auf dem transdermalen Weg in den Blutkreislauf eintreten. Feuchtigkeit, die aus den unter dem Stratum corneum liegenden Hautschichten stammt, kann dabei den Transport des Peptids durch die zumindest punktuell entfernten Abschnitte des Stratum corneums (d.h. durch die Mikrokanäle) erleichtern.

Mit Hilfe der zusätzlichen haftklebenden Schicht kann gegebenenfalls eine zusätzliche Fixierung des TTS auf der Haut erfolgen.

In einer besonderen Ausführungsform wird während der Anwendung der "Skin-ablation-Technik" das ablativ behandelte Hautareal farblich markiert, so dass das nachfolgende Anbringen des TTS exakt und leicht ausführbar ist.

Die Applikationsdauer einer Anwendung kann von wenigen (beispielsweise 2 bis 6) Stunden bis zu einem bis mehreren (beispielsweise 3 bis 7) Tagen erfolgen. Auch sind wiederholte Anwendungen möglich. Hierzu kann das TTS auf die ablativ behandelte Haut platziert werden, auf der bereits zuvor ein TTS appliziert gewesen ist. Vorzugsweise wird das TTS - insbesondere bei einer länger andauernden therapeutischen Anwendung - stets auf eine unmittelbar zuvor ablativ behandelte Hautfläche platziert.

Eine besondere Ausführungsform der Erfindung sieht vor, ein Transdermales Therapeutisches System (TTS), das
- eine Rückschicht, die mit einer haftklebenden Schicht ausgerüstet ist,
- eine FSH, eine Trägersubstanz in Form eines textilen Flächengebildes enthaltende Schicht, und
- eine Schutzfolie umfasst, im Rahmen einer Fertilitätstherapie zu verwenden.

Hierzu wird in einem ersten Schritt zunächst die Haut einer Patientin durch skin ablation-Technik mittels Laser ablativ behandelt. Dann wird in einem zweiten Schritt ein TTS mit einem GnRH-Agonisten (beispielsweise Leuprolid, Buserelin, Nafarelin, Histrelin, Goserelin oder Deslorelin, aber vorzugsweise Triptoreline) auf die so behandelte Hautstelle appliziert und für einen längeren Zeitraum (mindestens 12 Stunden, vorzugsweise 24 bis 48 Stunden) auf dieser Hautstelle belassen. Der dabei freigesetzte GnRH-Agonist wird transdermal an die Patientin abgegeben und führt zu einem Absinken des körpereigenen FSH-Levels dieser Patientin, der vorzugsweise während der Behandlung regelmäßig gemessen wird. Wenn der FSH-Level noch oberhalb des Zielwerts ist (der im Allgemeinen unterhalb von 10 mIE/mL liegt), werden diese ersten beiden Schritte wiederholt, jedoch an einer anderen Hautstelle.

Wenn der Zielwert des körpereigenen FSH-Levels der Patientin unterschritten ist, wird im dritten Schritt eine weitere Hautstelle der Patientin durch skin ablation-Technik mittels Laser ablativ behandelt. An diese Stelle wird nun im vierten Schritt das erfindungsgemäße TTS mit der Peptid FSH appliziert. Die transdermale Verabreichung von FSH bewirkt einen Anstieg des FSH-Levels der Patientin und die Bildung von Follikeln, was vorzugsweise durch Ultraschalluntersuchungen kontrolliert werden kann. In abschließenden Verfahrensschritten werden diese Follikel entnommen, in vitro befruchtet und der Patientin oder einer "Leihmutter" eingesetzt.

Das Verfahren zur Herstellung eines Transdermales Therapeutisches System (TTS) zur Verabreichung von Peptiden, welches eine wirkstoffhaltige Schicht enthält, die mindestens ein Peptid und mindestens eine Trägersubstanz für das Peptid enthält, umfasst mehrere Schritte.

In ersten Schritt wird das Peptid in Wasser gelöst, vorzugsweise in einen entsprechenden Puffer. Als besonders gut geeignete Lösungsmittel kommen isotonische Kochsalzlösung und wäßrige Puffer-Lösungen mit einem entsprechendem pH-Wert in Frage.

Weitere Hilfssubstanzen können dieser wirkstoffhaltigen Lösung zugegeben werden, wie z.B. Stabilisatoren und Konservierungsmittel (beispielsweise Mannitol, Cyclodextrine, Poloxamer (= Ethylenoxid-Propylenoxid-Blockcopolymere), Methionin, Histidin und deren Mischungen).

Konkret zählen zu den geeigneten, nicht-polymeren Hilfssubstanzen:
- mehrwertige Alkohole wie Threit, Erythrit, Pentaerythrit, Arabit, Adonit, Xylit, Sorbit, Mannit, Dulcit
- Monosaccharide wie Arabinose, Ribose, Xylose, Glucose, Mannose, Galactose, Fructose, Sorbose,
- Disaccharide wie Saccharose, Lactose, Maltose, Trehalose, Cellobiose,
- Oligosaccharide wie Raffinose,
- Cyclodextrine

Die so erhaltene Lösung wird in Form von Einzeldosen auf die in Form eines textilen Flächengebildes vorliegende Trägersubstanz aufgetragen. Die so erhaltene wirkstoffhaltige Schicht kann nun auf Rückschicht aufgelegt werden. In einer bevorzugten Ausführungsform wird die in Form eines textilen Flächengebildes vorliegende Trägersubstanz jedoch vor dem Auftragen der wirkstoffhaltigen Lösung auf die Rückschicht aufgelegt, insbesondere, wenn diese in Form eines "Überpflasters" (d.h. wenn die Rückschicht auf der, der wirkstoffhaltigen Schicht zugewandten Seite mit einem nicht-wasserlöslichen, haftklebenden Polymer beschichtet ist und eine Fläche besitzt, die größer ist als die Fläche der wirkstoffhaltigen Schicht) vorliegt.

In einen weiteren Arbeitsschritt wird der so erhaltene Verbund getrocknet, vorzugsweise bei Temperaturen unterhalb von 40°C, besonders bevorzugt unterhalb von 30°C, um die Lösemittel (und auch Wasser) zu entfernen, vorzugsweise bis zu einer gewünschten Restfeuchtigkeit von 0,5 bis 20%, vorzugsweise zwischen 1 und 10%.

Anschließend werden die einzeln dosierten TTS verpackt.

Die nachfolgenden Beispiele dienen der Veranschaulichung der Erfindung, ohne sie einzuschränken.

### Beispiel 1:

In Wasser gelöstes FSH wird mit einer wässrigen Phosphatpufferlösung (pH 7,0) gemischt. Zu dieser Lösung wird Cyclodextrin, Methionin, Poloxamer 188 und Metacresol zur Proteinstabilisierung gegeben. Diese Lösung wird dann auf ein textiles Flächengebilde (Vliesstoff), welches auf ein Überpflaster aufgelegt ist, flächengenau dosiert.

Die nachfolgende Tabelle gibt die Zusammensetzung der resultierenden wirkstoffhaltigen Schicht in getrocknetem Zustand wieder:

| wirkstoffhaltige Schicht | Menge [in mg] | Menge [in %] |
|---|---|---|
| FSH (900I.U.) | 0,111 | 0,48 |
| Cyclodextrin | 20,00 | 86,54 |
| Metacresol | 0,30 | 1,30 |
| Methionin | 0,10 | 0,43 |
| Poloxamer 188 | 0,10 | 0,43 |
| Natriummonodydrogenphosphat | 1,58 | 6,84 |
| Natriumdihydrogenphosphat | 0,92 | 3,98 |
| Summe: | **23,111** | **100,00** |

Als "Cyclodextrin" wird im konkreten Fall Hydroxypropyl-β-cyclodextrin verwendet. Bei dem Material der haftklebenden Schicht auf der Rückschicht handelt es sich um einen silikonbasierten Haftkleber mit einer Größe von 16.5cm², während die wirkstoffenthaltende Schicht eine Fläche von 5cm² aufweist.

Zusammen mit dem textilen Flächengebilde ergeben sich folgende Mengenverhältnisse:

| wirkstoffhaltige Schicht | Menge [in mg] | Menge [in %] |
|---|---|---|
| FSH (900I.U.) | 0,146 | 0,46 |
| Cyclodextrin | 20,00 | 63,2 |
| Metacresol | 0,30 | 0,95 |
| Methionin | 0,10 | 0,3 |
| Poloxamer 188 | 0,10 | 0,3 |
| Natriummonodydrogenphosphat | 1,58 | 5,0 |
| Natriumdihydrogenphosphat | 0,92 | 2,9 |
| textiles Flächengebilde | 8,5 | 26,86 |
| Summe: | **31,65** | **100,00** |

Zur Abdeckung der wirkstoffhaltigen Schicht und der überstehenden Ränder der Haftkleberschicht wird eine silikonisierte Polyesterfolie verwendet.

### Beispiel 2:

Muster gemäß Beispiel 1 werden hergestellt mit dem Unterschied, dass die Beladung mit FSH 300 I.U./5 cm², 600 I.U./5 cm² bzw. 1200 I.U./ 5 cm² entspricht.

### Beispiel 3:

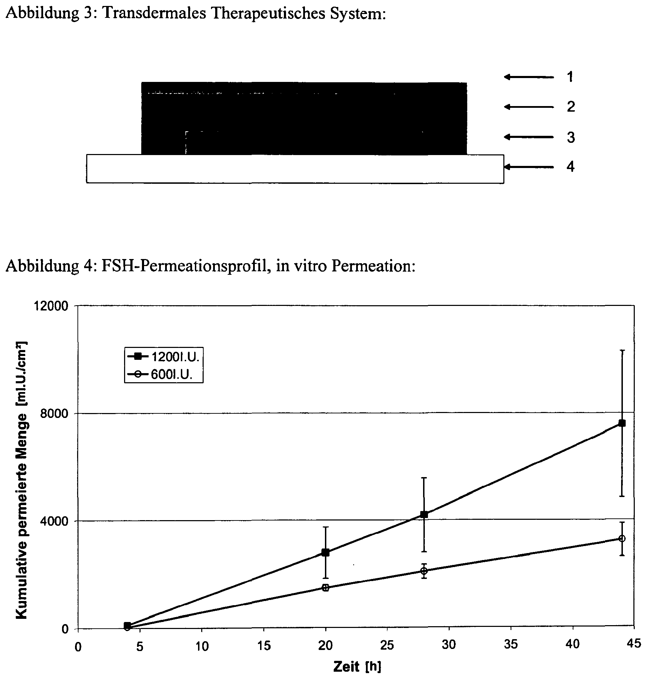

Muster der in Beispiel 2 hergestellten Transdermalen Therapeutischen Systeme mit FSH als Wirkstoff werden in einer Franz'schen Zelle hinsichtlich ihres Permeationsverhaltens durch mittels Laser vorbehandelte Kuheuterhaut untersucht. Die Ergebnisse dieser Untersuchungen sind in Abb. 4 dargestellt.

### Beschreibung der Abbildungen

Abbildung 1 zeigt den schematischen Aufbau intakter, normaler Haut mit einem vergößertern Ausschnitt der äußersten Schicht. Darin bedeuten:
   - E: = Epidermis
   - D: = Dermis
   - S: = Subcutis
   - B: = Blutgefäß
   - s.c.: = Stratum corneum
   - s.l.: = Stratum lucidum
   - s.gr.: = Stratum granulosum
   - s.sp.: = Stratum spinosum
   - s.b.: = Stratum basale
Abbildung 2 zeigt den schematischen Aufbau ablativ behandelter Haut. Hier bedeuten A das ablativ behandelte Hautareal und X die Flächen, an denen das Stratum corneum entfernt wurde.
Abbildung 3 zeigt den schematischen Aufbau eines Transdermalen Therapeutischen Systems gemäß Beispiel 1. Es bedeuten: 1 = Rückschicht, 2 = Haftkleberschicht, 3 = wirkstoffhaltige Schicht, 4 = Schutzfolie.
Abbildung 4 zeigt den Einfluss der FSH-Menge auf die in vitro-Permeation nach Beispiel 2. Als Permeationsbarriere für die Untersuchungen diente eine ablativ behandelte Humanhaut.

## Patentansprüche

1. Verwendung mindestens eines Peptids zur Herstellung eines Transdermalen Therapeutisches Systems (TTS), umfassend eine Rückschicht, die mit einer haftklebenden Schicht aus mindestens einem nicht-wasserlöslichen Polymer ausgerüstet ist, eine wirkstoffhaltige Schicht, die mindestens dieses Peptid und eine Trägersubstanz in Form eines textilen Flächengebildes enthält, und eine Schutzfolie, worin es sich bei dem Peptid um das follikelstimulierende Hormon (Follitropin, FSH) oder das somatotrope Hormon (Somatotropin, STH) handelt und die wirkstoffhaltige Schicht eine Fläche von 1 bis 100 cm², vorzugsweise von 2 bis 80 cm² sowie eine Dicke zwischen 10 und 200 µm, vorzugsweise zwischen 15 und 90 µm und besonders bevorzugt zwischen 20 und 80 µm aufweist,
zur Verabreichung dieses Peptids durch ablativ behandelte Haut an einen Patienten.

2. Verwendung nach Anspruch 1, **dadurch gekennzeichnet, dass** die Trägersubstanz nicht wasserlöslich ist.

3. Verwendung nach Anspruch 1 oder 2, **dadurch gekennzeichnet, dass** es sich bei dem Peptid um das follikelstimulierende Hormon (Follitropin, FSH) handelt.

4. Verwendung nach einem oder mehreren der Ansprüche 1 bis 3, **dadurch gekennzeichnet, dass** das Peptid in Form eines pharmazeutisch akzeptablen Salzes verwendet wird.

5. Verwendung nach einem oder mehreren der Ansprüche 1 bis 4, **dadurch gekennzeichnet, dass** das Peptid in einer Konzentration von 0,01 bis 99 Gew.-% (trocken), vorzugsweise zwischen 0,1 und 50 Gew.-% in der wirkstoffhaltigen Schicht enthalten ist.

6. Verwendung nach einem oder mehreren der Ansprüche 1 bis 5, **dadurch gekennzeichnet, dass** der Wassergehalt (Restfeuchtigkeit) der wirkstoffhaltigen Schicht unterhalb von 20%, vorzugsweise unterhalb von 10% des Gesamtgewichts dieser Schicht beträgt.

7. Verwendung nach einem oder mehreren der Ansprüche 1 bis 6, **dadurch gekennzeichnet, dass** die wirkstoffhaltige Schicht mindestens eine Substanz aus einer der Gruppe der nichtpolymeren Hilfssubstanzen, der Puffer, der Stabilisatoren und der Konservierungsmittel enthält.

8. Verwendung nach Anspruch 1, **dadurch gekennzeichnet, dass** das TTS nach einem Verfahren hergestellt wird, das die Schritte umfasst:
a. Auflegen einer in Form eines textilen Flächengebildes vorliegenden Trägersubstanz auf die mit einem nicht-wasserlöslichen, haftklebenden Polymer beschichtete Seite einer Rückschicht,
b. Herstellen einer wirkstoffhaltigen Lösung durch Mischen des Peptids mit Wasser und ggf. mindestens einer Hilfssubstanz,
c. Auftragen der wirkstoffhaltigen Lösung auf das textile Flächengebilde,
d. Trocknen bis zu einem vorbestimmten Wassergehalt unterhalb von 20%, vorzugsweise unterhalb von 10% unter Bildung der wirkstoffhaltigen Schicht, und
e. Abdecken des Verbunds aus Rückschicht und wirkstoffhaltiger Schicht mit einer Schutzfolie und Verpacken.

9. Verwendung nach Anspruch 8, **dadurch gekennzeichnet, dass** das Trocknen bei einer Temperatur unterhalb von 40°C erfolgt.

10. Verwendung nach Anspruch 1, wobei es sich bei dem Peptid um Follitropin handelt und bei dem Patienten um eine Person, welche eine assistierende Fertilitätstherapie benötigt.

11. Verwendung nach Anspruch 3 im Rahmen einer Fertilitätstherapie, bei welcher in einem ersten Schritt zunächst die Haut einer Patientin durch skin ablation-Technik mittels Laser ablativ behandelt wird, in einem zweiten Schritt ein TTS mit einem GnRH-Agonisten auf die so behandelte Hautstelle appliziert und für einen längeren Zeitraum auf dieser Hautstelle belassen wird und der dabei freigesetzte GnRH-Agonist transdermal an die Patientin abgegeben wird, in einem dritten Schritt eine weitere Hautstelle der Patientin durch skin ablation-Technik mittels Laser ablativ behandelt wird und an diese Stelle im vierten Schritt das TTS mit dem Wirkstoff FSH appliziert wird, um so FSH transdermal zu verabreichen und die Bildung von Follikeln in der Patientin zu bewirken.

## Claims

1. The use of at least one peptide for producing a transdermal therapeutic system (TTS) comprising a backing layer, which is furnished with a pressure-sensitively adhesive layer comprising at least one water-insoluble polymer; an active ingredient layer, which comprises at least this peptide and a carrier substance in the form of a sheetlike textile structure; and a protective sheet; wherein the peptide is follicle-stimulating hormone (follitropin, FSH) or somatotropic hormone (somatotropin, STH) and the active ingredient layer has an area of 1 to 100 cm², preferably of 2 to 80 cm², and a thickness of between 10 and 200 µm, preferably between 15 and 90 µm, and more preferably between 20 and 80 µm, for administering this peptide through ablatively treated skin to a patient.

2. The use as claimed in claim 1, wherein the carrier substance is not water-soluble.

3. The use as claimed in claims 1 or 2, wherein the peptide is follicle-stimulating hormone (follitropin, FSH).

4. The use as claimed in one or more of claims 1 to 3, wherein the peptide is used in the form of a pharmaceutically acceptable salt.

5. The use as claimed in one or more of claims 1 to 4, wherein the peptide is present in a concentration of 0.01 to 99 % by weight (dry), preferably between 0.1 and 50 % by weight in the active ingredient layer.

6. The use as claimed in one or more of claims 1 to 5, wherein the water content (residual moisture content) of the active ingredient layer is below 20 %, preferably below 10 % of the total weight of said layer.

7. The use as claimed in one or more of claims 1 to 6, wherein the active ingredient layer comprises at least one substance from one of the group of nonpolymeric auxiliaries, buffers, stabilizers, and preservatives.

8. The use as claimed in claim 1, wherein the TTS is produced by a method which comprises the steps of:
a. placing a carrier substance present in the form of a sheetlike textile structure onto that side of a backing layer that is coated with a water-insoluble, pressure-sensitively adhesive polymer,
b. preparing an active ingredient solution by mixing the peptide with water and optionally at least one auxiliary,
c. applying the active ingredient solution to the sheetlike textile structure,
d. drying to a predetermined water content of below 20 %, preferably below 10 %, to form the active ingredient layer, and
e. lining the assembly composed of backing layer and active ingredient layer with a protective sheet, and packaging it.

9. The use as claimed in claim 8, wherein the drying takes place at a temperature below 40 °C.

10. The use as claimed in claim 1, where the peptide is follitropin and the patient is a person in need of assistive fertility therapy.

11. The use as claimed in claim 3 in the context of a fertility therapy in which first of all, in a first step, the skin of a female patient is ablatively treated by a laser skin ablation technique; in a second step a TTS with a GnRH agonist is applied to the skin location thus treated and is left at this skin location for a prolonged time period, and the GnRH agonist released in this operation is delivered transdermally to the patient; in a third step, a further skin location of the patient is ablatively treated by a laser skin ablation technique, and at this location, in the fourth step, the TTS with the active ingredient FSH is applied, in order thus to administer FSH transdermally and to bring about the formation of follicles in the patient.

## Revendications

1. Utilisation d'au moins un peptide pour la réalisation d'un système thérapeutique transdermique (STT), comprenant une couche arrière qui est équipée d'une couche semi-adhésive à base d'au moins un polymère insoluble dans l'eau, une couche incluant une substance active qui contient au moins ce peptide et un véhicule sous la forme d'une structure plane textile, et un film protecteur, dans lequel le peptide est l'hormone de stimulation folliculaire (follitropine, FSH) ou l'hormone somatotrope (somatotropine, STH) et la couche contenant une substance active présente une superficie de 1 à 100 cm², de préférence de 2 à 80 cm², ainsi qu'une épaisseur comprise entre 10 et 200 µm, de préférence comprise entre 15 et 90 µm et le plus préférentiellement comprise entre 20 et 80 µm, pour l'administration de ce peptide à un patient à travers une peau traitée par ablation.

2. Utilisation selon la revendication 1, **caractérisée en ce que** le véhicule est insoluble dans l'eau.

3. Utilisation selon la revendication 1 ou 2, **caractérisée en ce que** le peptide est l'hormone de stimulation folliculaire (follitropine, FSH).

4. Utilisation selon l'une ou plusieurs des revendications 1 à 3, **caractérisée en ce que** le peptide est utilisé sous la forme d'un sel pharmaceutiquement acceptable.

5. Utilisation selon l'une ou plusieurs des revendications 1 à 4, **caractérisée en ce que** le peptide est contenu dans une concentration de 0,01 à 99 % en poids (sec), de préférence entre 0,1 et 50 % en poids dans la couche contenant une substance active.

6. Utilisation selon l'une ou plusieurs des revendications 1 à 5, **caractérisée en ce que** la teneur en eau (humidité résiduelle) de la couche contenant une substance active se situe en dessous de 20 %, de préférence en dessous de 10 % du poids total de cette couche.

7. Utilisation selon l'une ou plusieurs des revendications 1 à 6, **caractérisée en ce que** la couche contenant une substance active contient au moins une substance issue de l'un parmi le groupe des substances auxiliaires non polymères, des tampons, des stabilisateurs et des agents de conservation.

8. Utilisation selon la revendication 1, **caractérisée en ce que** le STT est réalisé selon un procédé qui comprend les étapes :
a. de pose d'un véhicule présent sous la forme d'une structure plane textile sur la face revêtue d'un polymère insoluble dans l'eau et semi-adhésif d'une couche arrière,
b. de réalisation d'une solution contenant une substance active par mélange du peptide avec de l'eau et le cas échéant d'au moins une substance auxiliaire,
c. d'application de la solution contenant une substance active sur la structure plane textile,
d. de séchage jusqu'à une teneur en eau prédéterminée en dessous de 20 %, de préférence en dessous de 10 % lors de la formation de la couche contenant une substance active, et
e. de recouvrement du composite à base de la couche arrière et de la couche contenant une substance active avec un film protecteur et de conditionnement.

9. Utilisation selon la revendication 8, **caractérisée en ce que** le séchage est effectué à une température en dessous de 40 °C.

10. Utilisation selon la revendication 1, dans laquelle le peptide est la follitropine et le patient est une personne qui a besoin d'un traitement d'assistance contre l'infertilité.

11. Utilisation selon la revendication 3 dans le cadre d'un traitement contre l'infertilité, dans lequel lors d'une première étape la peau d'une patiente est tout d'abord traitée par ablation au moyen d'un laser par une technique d'ablation de la peau, lors d'une deuxième étape un STT est appliqué avec un agoniste de la GnRH sur la zone cutanée ainsi traitée et laissé sur cette zone cutanée pendant un laps de temps plus long et l'agoniste de la GnRH libéré à cette occasion est transmis par voie transdermique à la patiente, lors d'une troisième étape une autre zone cutanée de la patiente est traitée par ablation au moyen d'un laser par une technique d'ablation de la peau et à cet endroit lors d'une quatrième étape le STT est appliqué avec la substance active FSH pour ainsi administrer la FSH par voie transdermique et induire la formation de follicules chez la patiente.
